(19) Europäisches Patentamt
European Patent Office
Office européen des brevets

(11) Veröffentlichungsnummer : **0 388 417 B1**

(12) # EUROPÄISCHE PATENTSCHRIFT

(45) Veröffentlichungstag der Patentschrift :
**19.02.92 Patentblatt 92/08**

(51) Int. Cl.$^5$ : **A61K 7/06**

(21) Anmeldenummer : **88909493.4**

(22) Anmeldetag : **04.11.88**

(86) Internationale Anmeldenummer :
**PCT/AT88/00090**

(87) Internationale Veröffentlichungsnummer :
**WO 89/04162 18.05.89 Gazette 89/11**

(54) **HAARWUCHSMITTEL.**

(30) Priorität : **06.11.87 AT 2932/87**

(43) Veröffentlichungstag der Anmeldung :
**26.09.90 Patentblatt 90/39**

(45) Bekanntmachung des Hinweises auf die Patenterteilung :
**19.02.92 Patentblatt 92/08**

(84) Benannte Vertragsstaaten :
**AT BE CH DE FR GB IT LI LU NL SE**

(56) Entgegenhaltungen :
**DE-A- 1 467 946**
**US-A- 3 463 862**

(73) Patentinhaber : **LEBENSAFT, Walter, Dr.**
**Endemanngasse 6-18/12**
**A-1238 Wien (AT)**

(72) Erfinder : **LEBENSAFT, Walter, Dr.**
**Endemanngasse 6-18/12**
**A-1238 Wien (AT)**

(74) Vertreter : **Brauneiss, Leo et al**
**Patentanwälte Dipl.-Ing. Leo Brauneiss, Dipl.-Ing. Dr. Helmut Wildhack Landstrasser Hauptstrasse 50 Postfach 281**
**A-1031 Wien (AT)**

EP 0 388 417 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung bezieht sich auf ein Haarwuchsmittel zur Applikation auf die menschliche Kopfhaut.

Bekanntlich gibt es eine Vielzahl von Haarwuchsmitteln mit unterschiedlichen Wirkstoffen. Ebenso bekannt ist aber auch, daß die Versagerquote relativ hoch ist, d.h. bei einer Vielzahl von Kahlköpfigen bleiben die bekannten Haarwuchsmittel wirkungslos.

Die Erfindung setzt sich zur Aufgabe, ein neues Haarwuchsmittel zu schaffen, welches diesen Nachteil vermeidet, d.h. eine Haarwuchsanregung zumindest bei einigen jener Personen bringt, bei welchen die bisher bekannten Mittel versagten. Die Erfindung löst diese Aufgabe dadurch, daß das Haarwuchsmittel einen Gehalt an Epichlorhydrin in homöopathischer Verdünnung hat, wobei das Volumenverhältnis von Epichlorhydrin jum Verdünnungsmittel 1:100 bis 1000000, vorzugsweise 1:1000 bis 1:10000, beträgt.

Epichlorhydrin (genauer: $\alpha$ -Epichlorhydrin) ist an sich bekannt, es handelt sich um 1-Chlor-2.3-epoxypropan mit der Strukturformel

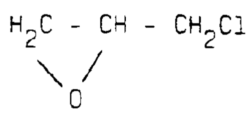

Diese Substanz ist an sich giftig, in der homöopathischen Verdünnung, in welcher sie im Sinne der Erfindung zur Anwendung auf die menschliche Kopfhaut gelangt, jedoch unbedenklich. Vielmehr hat es sich gezeigt, daß durch das erfindungsgemäße Haarwuchsmittel bei Anwendung auf der Kopfhaut eine Verstäkrung des bestehenden Haarwuchses bzw. eine Neubildung von Haarzellen und somit ein neuer Haarwuchs bei Kahlheit erzielt werden kann.

Im Rahmen der Erfindung ist das Verdünnungsmittel zweckmäßig Ethylalkohol und bzw. oder destilliertes Wasser. Das bevorzugte Verdünnungsmittel ist Ethylalkohol, da Epichlorhydrin darin besser mischbar ist als in Wasser. Das Verdünnungsverhältnis beträgt mit Rücksicht auf den angestrebten homöopathischen Verdünnungsgrad (volumensmäßig berechnet) 1:100 bis 1:1 000 000 gesteigert werden. Bevorzugte Verdünnungswerte liegen zwischen 1:1 000 und 1: 10 000.

Das Haarwuchsmittel kann gemäß einer Weiterbildung der Erfindung auch einen Gehalt an Eiweißstoffen, insbesondere Albumin, haben. Die Verwendung von Eiweißstoffen in Haarwuchsmitteln ist an sich bekannt (US-PS 3 463 862).

Es ist bekannt, Epichlorhydrin zum Schutz vor Haaren von Umwelteinflüssen und vor Licht einzusetzen (DE-OS 3 718 496) oder einer Stoffmischung zur Kaltwellenbehandlung von Haaren beizusetzen (DE-PS 1 467 946). Eine Anregung auf die Erfindung konnte man aus diesen Literaturstellen nicht entnehmen, weil bei den bekannten Verwendungen Epichlorhydrin die Rolle einer mechanischen Schützsubstanz übernimmt, welche sich verfestigt und dadurch das Haar durch Bildung einer Schutzschicht schützt bzw. durch die Verfestigung stützt. Im Sinne der Erfindung tritt jedoch Epichlorhydrin durch die Aufbringung auf die menschliche Haut bzw. durch die percutane Aufnahme in den Körper - wenn auch nur in homöopathischen Mengen - ein und tritt mit den Körpersubstanzen in chemische Wechselwirkung. Die diesbezüglichen Vorgänge sind noch ungeklärt.

Bei der Durchführung einer Kopfhautbehandlung mit dem erfindungsgemäßen Haarwuchsmittel wird beispielsweise wie folgt vorgegangen:

Das Haar wird gewaschen und getrocknet. Sodann wird die Kopfhaut mit geringen Mengen des Haarwuchsmittel mehrmals eingerieben. Das Haarwuchsmittel hat als Wirkstoff Epichlorhydrin, welches in Ethylalkohol in einer Verdünnung von 1:5 000 (volumgsmäßig berechnet) vorliegt. Das Haarwuchsmittel enthält keine sonstigen Bestandteile. Zur Durchführung der Einreibung genügt es, den Finger in das Haarwuchsmittel einzutauchen und an der Kopfhaut zu reiben und diesen Vorgang mehrmals zu wiederholen. Die hiebei auf die Kopfhaut applizierte Menge an Haarwuchsmittel beträgt etwa 1 ml pro Behandlung. Das Verdünnungsmittel wird auf der Kopfhaut trocknen bzw. verdunsten gelassen. Diese Vorgänge werden in Abständen von etwa 14 Tagen wiederholt. Nach wiederholter Behandlung ergibt sich eine Verstärkung bzw. Neubildung des Haarwuchses und einer Regeneration der Kopfhaut.

## Patentansprüche

1. Haarwuchsmittel zur Applikation auf die menschliche Kopfhaut, gekennzeichnet durch einen Gehalt an Epichlorhydrin wobei das Volumenverhältnis von Epichlorhydrin zum Verdünnungsmittel 1:100 bis 1:1 000 000, vorzugsweise 1:1 000 bis 1:10 000, beträgt.

2. Haarwuchsmittel nach Anspruch 1, dadurch gekennzeichnet, daß das Verdünnungsmittel Ethylalkohol und bzw. oder destilliertes Wasser ist.

3. Haarwuchsmittel nach einem der Ansprüche 1 bis 3, dadurch gekennzeichnet, daß es auch einen Gehalt an Eiweißstoffen, insbesondere Albumin, hat.

## Claims

1. Hair growth promoter for application to the human scalp, comprising epichlorhydrin, wherein the volume ratio of epichlorhydrin to thinner is 1:100 to 1:1,000,000, and preferably 1:1,000 to 1:10,000.

2. Hair growth promoter according to claim 1, wherein the thinner comprises ethyl alcohol and/or distilled water.

3. Hair growth promoter according to claim 1, further comprising proteins, in particular albumin.

## Revendications

1. Produit capillaire pour l'application au cuir chevelu humain, caractérisé par une teneur en épichlorhydrine, le rapport volumétrique de l'épichlorhydrine au diluant étant de 1 : 100 à 1 : 1000000, de préférence de 1 : 1000 à 1 : 10000.

2. Produit capillaire selon la revendication 1, caractérisé en ce que le diluant est l'alcool éthylique et/ou l'eau distillée.

3. Produit capillaire selon une des revendications 1 à 3, caractérisé en ce qu'il contient aussi des proteïnes, en particulier l'albumine.